# EUROPEAN PATENT APPLICATION

(11) **EP 2 111 832 A1**
(43) Date of publication of application: **28.10.2009**
(21) Application number: 07859825.7
(22) Date of filing: 14.12.2007
(51) Int. Cl.: A61F 13/15, A61F 5/44, A61F 13/49, A61F 13/494

(54) **DISPOSABLE DIAPER**

(30) Priority: 09.02.2007 JP 2007031231
(71) Applicant: Uni-charm Corporation, Ehime 799-0111 (JP)
(72) Inventor: MINATO, Hironao, Kanonji-shi Kagawa 769-1602 (JP); NAKAJIMA, Kaiyo, Kanonji-shi Kagawa 769-1602 (JP); TAKADA, Naoko, Kanonji-shi Kagawa 769-1602 (JP)
(74) Representative: Stark, Gordon Drummond
(86) International application number: PCT/JP2007/074095
(87) International publication number: WO 2008/096506

(57) **Abstract**

The present invention aims to provide a disposable diaper improved so that, when the diaper is put on a wearer's body, an area of the wearer's skin defined between his or her external genital and anus can reliably protected from being soiled with body waste. The disposable diaper is provided on the inner surface of a liquid-pervious inner sheet 32 with a separator sheet 33b formed with a front opening 38 and a rear opening 39. The separator sheet 33 has end regions 34, 36 opposite in a front-back direction A both bonded to the inner sheet 32 and a middle region 35 left free from the inner sheet 32. The separator sheet 33b further includes elastic members 37_{L}, 38_{R} extending under tension in the front-back direction A along inner edges of the front and rear openings 38, 39. The separator sheet 33b is bonded along lateral zones 48_{L}, 48_{R} thereof to the inner sheet 32. Between the respective lateral zones 48_{L}, 48_{R} and a center line P-P, the separator sheet 33b is provided with means assisting the middle region 35 to get near to the diaper wearer's skin.

## Description

### TECHNICAL FIELD

The present invention relates to a disposable diaper.

### RELATED ART

In disposable diapers, it is known, for example, from JP 61-41304 A (PATENT DOCUMENT 1) and JP 2002-11044 A (PATENT DOCUMENT 2) to interpose a separator sheet between a bodily fluid-absorbent structure and the wearer's skin.

In the case of the disposable diaper disclosed in PATENT DOCUMENT 1, a hydrophobic inner sheet is provided above a cover sheet for an absorbent pad structure. This inner sheet is formed in a crotch region with an opening for passage of body waste and transversely opposite lateral zones of the opening are respectively provided with elastic bands extending under tension in the front-back direction. When this diaper is put on the wearer's body, contraction of the elastic bands causes the inner sheet to be spaced upward from the absorbent pad and to come in contact with the wearer's skin so as to prevent the absorbent pad from coming in direct contact with the wearer's skin.

The diaper disclosed in PATENT DOCUMENT 2 is of pant-type and provided above a liquid-pervious inner sheet covering an absorbent structure with a skin-contact sheet. This skin-contact sheet has longitudinally opposite end regions bonded to the inner sheet or an outer covering sheet and is formed in the crotch region thereof with an opening for passage of body waste. Elastic members are attached under tension to the skin-contact sheet so as to surround the opening.
PATENT DOCUMENT 1: JP 61-41304 A
PATENT DOCUMENT 2: JP 2002-11044 A

### DISCLOSURE OF THE INVENTION

### PROBLEM TO BE SOLVED BY THE INVENTION

In the case of the diaper disclosed in PATENT DOCUMENT 1, the inner sheet is merely formed with the opening within which the external genital and the anus are uncovered. With such diaper, the area of the wearer's skin defined between the external genital and the anus might be in contact with the cover sheet wetted with bodily fluids for a long period and develop red rash. In addition, loose passage might flow on the wearer' s skin toward the external genital and soil this.

The opening in the diaper disclosed in PATENT DOCUMENT 2 may be sometimes liable to become narrower under the effect of the elastic members surrounding the opening under tension. In consequence, higher the extension percentage of the elastic members, the width of the opening may become unacceptably narrower for passage of body waste. The area of the skin-contact sheet designed to cover the area of the wearer's skin defined between the external genital and the anus extends so as to cover the periphery of the anus and the transversely central region of the rear waist region. Consequentially, if this sheet is caught by the cleft of the wearer's buttock, the opening will be substantially closed and it will be impossible for the skin-contact sheet to fulfill the expected function thereof.

It is a principal object of the present invention to solve the various problems left behind unsolved by diapers of prior art.

### MEASURE TO SOLVE THE PROBLEM

The object set forth above is achieved, according to the present invention, by an improvement in the disposable diaper including a crotch region having a front-back direction and a width direction, a front waist region extending forward from the crotch region and a rear waist region extending rearward from the crotch region, the crotch region comprising a liquid-pervious inner sheet, a liquid-impervious outer covering sheet and a bodily fluid-absorbent core sandwiched between these two sheets, the disposable diaper further including a separator sheet extending in the front-back direction on the inner side of the inner sheet and formed in a front region thereof with a front opening for passage of body waste and in a rear region thereof with a rear opening for passage of body waste, the separator sheet having end regions opposite in the front-back direction being bonded to the inner sheet in the front waist region and the rear waist region, respectively, and the separator sheet including a middle region defined between the front opening and the rear opening and adapted to be spaced upward from the inner sheet in the crotch region so as to prevent the inner sheet from coming in contact with a diaper wearer's skin.

The improvement according to the present invention is characterized by features as follow: The separator sheet has lateral zones extending in the front-back direction and bonded to the inner sheet. The separator sheet is provided on each side of a center line bisecting a width of the diaper with at least a single elastic member attached thereto under tension so as to extend along respective associated inner edges of the front opening and the rear opening in the front-back direction and to bow in the middle region toward the center line. The separator sheet is further formed along the lateral zones in the middle region or in areas defined between the lateral zones and the center line with means adapted to assist a movement of the middle region on the center line toward the diaper wearer's skin under contraction of the elastic members.

According to one preferred embodiment of the present invention, the assisting means are formed by leaving segments of respective the lateral zones of the separator sheet extending in the middle region not bonded to the inner sheet.

According to another preferred embodiment of the present invention, the assisting means are formed by providing the separator sheet with slits formed between respective the lateral zones and the center line, respectively, so as to extend in the front-back direction.

According to still another preferred embodiment of the present invention, the front opening of the separator sheet is cut out from the separator sheet in the front region so as to describe a U-shape opening forward while the rear opening of the separator sheet is cut out from the separator sheet in the rear region so as to describe a U-shape opening rearward.

### EFFECT OF THE INVENTION

In the case of the disposable diaper according to the present invention, the diaper may be put on the wearer's body in a manner that the middle region of the separator sheet is positioned between the wearer's external genital and anus, the wearer' external genital is uncovered within the front opening and the wearer's anus is uncovered within the rear opening to assure that the contractile force of the elastic members cooperate with the assisting means to facilitate the middle region to come in contact with the area of the wearer's skin defined between the external genital and the anus. As a consequence, the wearer's skin can be prevented from coming in contact with the inner sheet wetted with bodily fluids and/or soiled with loose passage. Both the front opening and the rear opening of the separator sheet are formed to describe U-shape and the inner edges of the respective openings are provided with the elastic members under tension. In this way, these openings would not be closed even if the middle region is caught by the cleft of the wearer's buttock.

The means facilitating the middle region to get near to the wearer's skin may formed by leaving segments of respective the lateral zones of the separator sheet extending in the middle region not bonded to the inner sheet or providing the separator sheet with the slits.

According to the embodiment of the present invention wherein the front opening of the separator sheet is cut out from the separator sheet in the front region so as to describe a U-shape opening forward and the rear opening of the separator sheet is cut out from the separator sheet in the rear region so as to describe a U-shape opening rearward, these openings would not be closed even if the middle region of the separator sheet is caught by the cleft of the wearer's buttock.

### BRIEF DESCTIPTION OF THE DRAWINGS

[FIG. 1] Fig. 1 is a partially cutaway perspective view of a disposable diaper.
[FIG. 2] Fig. 2 is a sectional view taken along the line II-II in Fig. 1.
[FIG. 3] Fig. 3 is a plan view showing the diaper of Fig. 1 as has been flatly developed.
[FIG. 4] Fig. 4 is an exploded perspective view illustrating how the diaper of Fig. 3 is assembled.
[FIG. 5] Fig. 5 is a scale-enlarged perspective view showing a bodily fluid-absorbent structure as partially broken away.
[FIG. 6] Fig. 6 is a perspective view showing one exemplary embodiment of the bodily fluid-absorbent structure.
[FIG. 7] Fig. 7 is a sectional view taken along the line VII-VII in Fig. 6.
[FIG. 8] Fig. 8 is a sectional view taken along the line VIII-VIII in Fig. 6.
[FIG. 9] Fig. 9 is a view similar to Fig. 5, showing another exemplary embodiment of the bodily fluid-absorbent structure.

### IDENTIFICATION OF REFERENCE NUMERALS USED IN THE DRAWINGS

- 1: disposable diaper
- 7: outer covering sheet
- 11: crotch region
- 12: front waist region
- 13: rear waist region
- 31: core
- 32: inner sheet
- 33b: separator sheet
- 35: middle zone
- 37, 37_{L}, 37_{R}: elastic member
- 38: front opening
- 39: rear opening
- 48, 48_{L}, 48_{R}: lateral zone
- 71_{L} 71_{R}: inner edge
- 73_{L}, 73_{R}: inner edge
- 76_{L}, 76_{R}: lateral zone in middle region
- 87_{L}, 87_{R}: slit (means assisting a movement of middle region)
- A: front-back direction
- B: width direction
- P-P: center line (longitudinal center line)

### DETAILED DESCRIPTION OF THE PREFERRED EMBODIMENTS

The present invention will be described in more details with reference to the accompanying drawings.

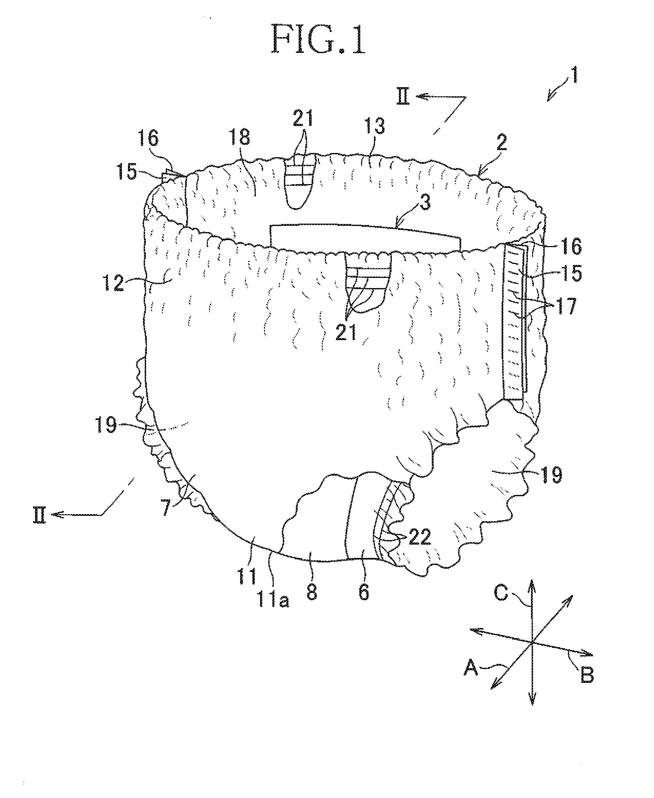

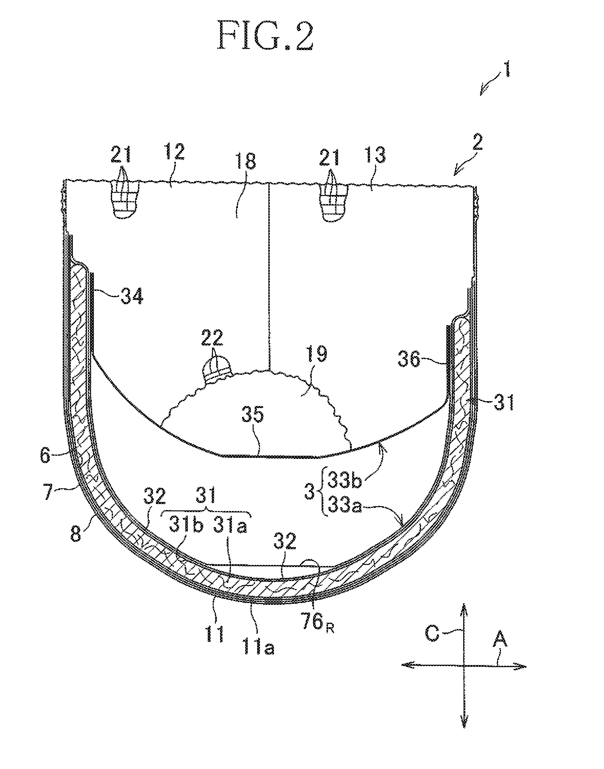

Fig. 1 is a partially cutaway perspective view of a diaper 1 and Fig. 2 is a sectional view taken along the line II-II in this perspective view. The diaper 1 is shown by Fig. 1 as it is put on the wearer's body, wherein a front-back direction, a width direction and a height direction are designated by A, B, C, respectively, and wherein the diaper 1 has a pant-shape covering chassis 2 and a bodily fluid-absorbent structure 3 attached to an inner side of the covering chassis 2. The covering chassis 2 comprises an inner covering sheet 6, an outer covering sheet 7 and a liquid-impervious leak-barrier sheet 8 sandwiched between these inner and outer covering sheets 6, 7 so as to define a crotch region 11, a front waist region 12 extending forward from the crotch region 11 and a rear waist region 13 extending rearward from the crotch region 11. The front and rear waist regions 12, 13 respectively have transversely opposite lateral zones 15, 15, 16, 16 put flat and fused together at a plurality of fusion segments 17 arranged intermittently in the height direction C indicated in Fig. 1. Thereupon, a waist-opening 19 is formed and, at the same time, the front and rear waist regions 12, 13 cooperate with the crotch region 11 to form a pair of leg-openings 19. Along respective peripheries of the waist-opening 18 and the leg-openings 19, a plurality of waist elastic members 21 and leg elastic members 22 are sandwiched between the inner covering sheet 6 and the outer covering sheet 7 and bonded under tension to at least one of these inner and outer covering sheets 6, 7. The bodily fluid-absorbent structure 3 is attached to the covering chassis 2 at least in the crotch region 11 and basically comprises a liquid-absorbent panel 33a and a separator sheet 33b. The liquid-absorbent panel 33a, in turn, comprises a bodily fluid-absorbent core 31 formed by liquid-absorbent material 31a wrapped with tissue paper 31b and a liquid-absorbent inner sheet 32 adapted to cover at least the side of the core 31 facing the wearer's skin. The separator sheet 33b is provided further nearer to the wearer's skin than the inner sheet 32. Referring to Fig. 2, the separator sheet 33b has a front end zone 34 and a rear end zone 36 both bonded to the inner sheet 32 in the front and rear waist regions 12, 13, respectively, and a middle region 35 spaced upward from the inner sheet 32 in the crotch region 11. Referring also to Fig. 2, the core 31 is sandwiched between the inner sheet 32 and the leak-barrier sheet 8 so as to be covered from the outer side with this leak-barrier sheet 8. With this arrangement, any quantity of bodily fluids once absorbed by the core 31 would not leak out from the diaper 1.

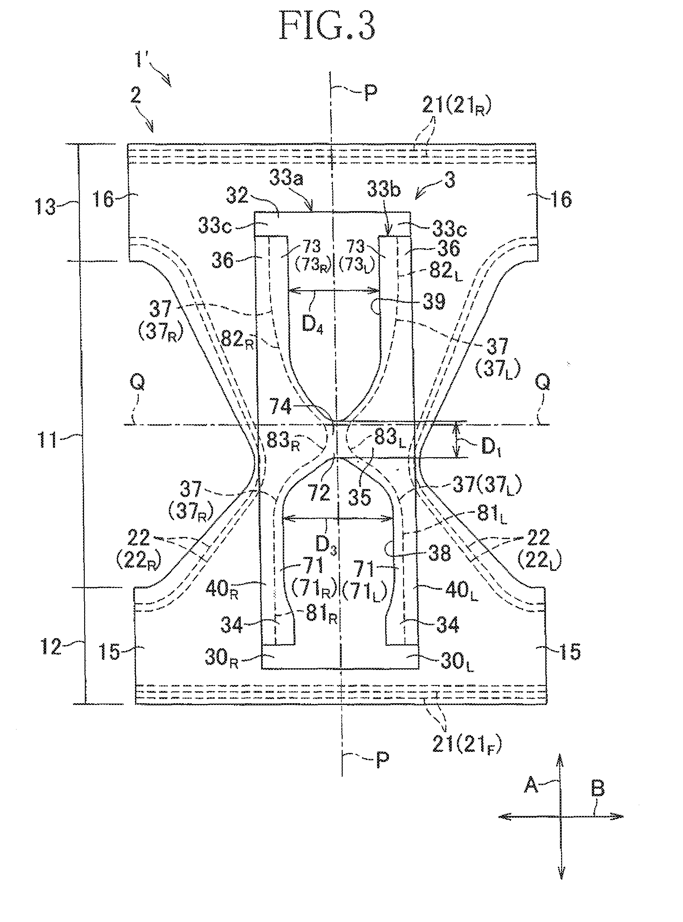

Fig. 3 is a plan view showing the diaper 1' after the front and rear waist regions 12, 13 have been peeled apart at the fusion segments 17 and then developed in the front-back direction A and the width direction B of the diaper 1, wherein the waist elastic members 21 consisting of front and rear waists elastic members 21_{F}, 21_{R}, the leg elastic members 22 consisting of left and right legs elastic members 22_{L}, 22_{R} and crotch region elastic members 37 consisting of left and right crotch region elastic members 37_{L}, 37_{R} attached under tension to the separator sheet 33b are respectively indicated by chain lines. It should be appreciated here that the terms "left" and "right" used herein mean the left and right hands for the wearer of the diaper 1 and the diaper 1' is bilaterally symmetric about a longitudinal center line P-P bisecting the width of the diaper 1'. The diaper 1' also has a transverse center line Q-Q bisecting a length of the diaper 1' measured as in the front-back direction A. As will be apparent from Fig. 3, the covering chassis 2 presents a concave shape curved inwardly and the bodily fluid-absorbent structure 3 presents a rectangular shape. The separator sheet 33b in the bodily fluid-absorbent structure 3 has a pair of lateral zones 40_{L}, 40_{R} and the middle region 35 serving to connect these lateral zones 40_{L}, 40_{R} so that these lateral zones and the middle zone cooperate together to define a U-shaped front opening 38 extending from the crotch region 11 toward the front waist region 12 and a U-shaped rear opening 39 extending from the crotch region 11 toward the rear waist region 13. The front opening 38 has transversely opposite inner edges 71_{L}, 71_{R} extending in the front-back direction A and generically referred to as lateral edges 71 and a curved bottom edge 72 connecting these opposite inner edges 71_{L}, 71_{R}. Similarly, the rear opening 39 has transversely opposite inner edges 73_{L}, 73_{R} extending in the front-back direction A and generically referred to as lateral edges 73 and a curved bottom edge 74 connecting these opposite inner edges 73_{L}, 73_{R}.

The diaper 1 of Fig. 1 is put on the wearer's body so that the front opening 38 shown in Fig. 3 may uncover the wearer's external genital therein, the rear opening 39 would uncover the wearer's anus therein and the middle region 35 may be held in contact with the wearer' s skin between the external genital and the anus. To achieve such desired position relationship, the bottom edge 72 of the front opening 38 preferably lies ahead of the transverse center line Q-Q and the bottom edge 74 of the rear opening 39 preferably lies on the transverse center line Q-Q or in the vicinity of the transverse center line Q-Q whether aside forward or rearward from this center line Q-Q.

Referring to Fig. 3, each of the crotch region elastic members 37_{L}, 37_{R} is preferably formed by a single rubber-thread and extends in the front-back direction A along the associated lateral zone of the front opening 38 and the rear opening 39. These elastic members 37_{L}, 37_{R} contract in the front-back direction A as the crotch region 11 of the diaper 1 is bowed in the front-back direction A as seen in Figs. 1 and 2. In consequence, a dimension of the separator sheet 33b as measured in the front-back direction A in Fig. 3 is reduced so as to space the middle region 35 upward from the inner sheet 32 (See Fig. 2).

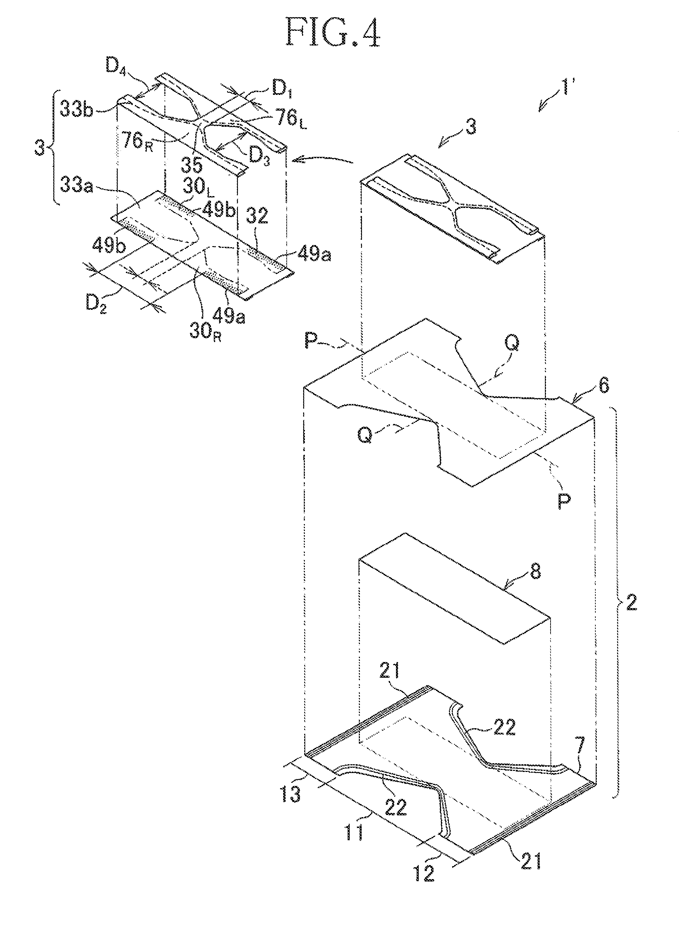

Fig. 4 is an exploded perspective view illustrating how the diaper 1' is assembled. The outer covering sheet 7 is formed by non-woven fabric or plastic film and provided on its inner surface with the waist elastic members 21 and the leg elastic members 22 attached under tension thereto by hot melt adhesive (not shown). In addition to these elastic members 21, 22, the leak-barrier sheet 8 formed by liquid-impervious plastic film is adhesion- or fusion-bonded to the inner surface of the outer covering sheet 7. To the respective inner surfaces (i.e., upper surfaces as viewed in Fig. 4) of the outer covering sheet 7 and the leak-barrier sheet 8, the inner covering sheet 6 which is same as the outer covering sheet 7 in shape as well as in size and formed by non-woven fabric or plastic film is adhesion- or fusion-bonded. The bodily fluid-absorbent structure 3 is bonded to the inner surface of the inner covering sheet 6 by hot melt adhesive (not shown). The bodily fluid-absorbent structure is bonded, substantially over the entire outer surface (i.e., bottom surface as viewed in Fig. 4) of the liquid-absorbent panel 33a to the inner covering sheet 6 and the separator sheet 33b is bonded to the inner surface of the liquid-absorbent panel 33a along lateral zones 30_{L}, 30_{R} of the liquid-absorbent panel 33a.

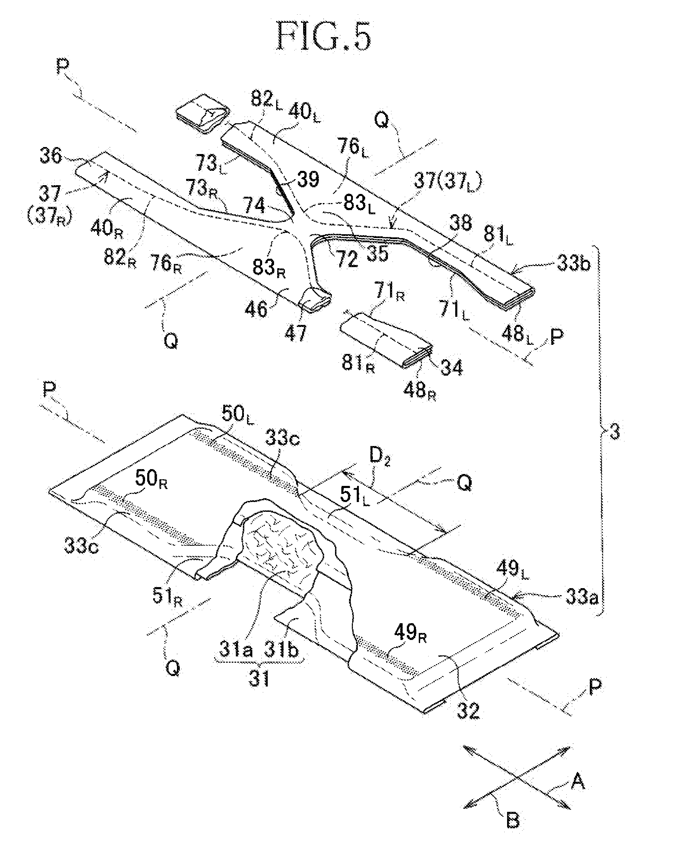

Fig. 5 is a scale-enlarged perspective view showing the bodily fluid-absorbent structure 3 illustrated in the exploded perspective view of Fig. 4 as partially broken away. The liquid-absorbent panel 33a in the bodily fluid-absorbent structure 3 comprises the core 31 formed, for example, by fluff pulp or a mixture of fluff pulp and super-absorbent polymer particles wrapped with the tissue paper 31b and the liquid-pervious inner sheet 32 covering the inner surface, the lateral surface and at least a part of the outer surface of the core 31. In Fig. 5, the longitudinal center line P-P and the transverse center line Q-Q of the diaper 1' are indicated to clarify the directional character of this liquid-absorbent panel 33a. The separator sheet 33b is formed by a pair of non-woven fabric layers 46, 47 put flat together and sandwich therebetween the crotch region elastic members 37_{L}, 37_{R} extending in bilaterally symmetric relationship about the longitudinal center line P-P. The crotch region elastic member 37_{L} includes a first segment 81_{L} extending substantially in a linear fashion along the inner edge 71_{L} of the front opening 38, a second segment 82_{L} extending substantially in a linear fashion along the inner edge 73_{L} of the rear opening 39 and a third segment 83_{L} extending along the bottom edges 72, 74 in the middle region 35 so as to be convex toward the longitudinal center line P-P. Similarly, the other crotch region elastic member 37_{R} includes a first segment 81_{R} extending substantially in a linear fashion along the inner edge 71_{R} of the front opening 38, a second segment 82_{R} extending substantially in a linear fashion along the inner edge 73_{R} of the rear opening 39 and a third segment 83_{R} extending along the bottom edges 72, 74 in the middle region 35 so as to be convex toward the longitudinal center line P-P. In this manner, these elastic members 37_{L}, 37_{R} extend along the respective lateral edges of the front and rear openings 38, 39 in the front-back direction A so as to get near toward each other in the middle region 35 and are bonded under section to at least one of the non-woven fabric layers 46, 47. Respective lateral zones 48_{L}, 48_{R} of the lateral zones 40_{L}, 40_{R} are folded back toward the longitudinal center line P-P and bonded to the inner sheet 32 of the liquid-absorbent panel 33a along adhesive regions 49_{L}, 50_{L} and 49_{R}, 50_{R}, each illustrated as a plurality of dots. It should be noted here that lateral zones 76_{L}, 76_{R} of the middle region 35 making parts of the respective lateral zones 48_{L}, 48_{R} are left free from the inner sheet 32. In other words, between the adhesive regions 49_{L}, 50_{L} and between the adhesive regions 49_{R}, 50_{R} as viewed in the front-back direction A of the diaper 1, non-adhesive regions 51_{L}, 51_{R} for the separator sheet 33b each having a length D₂ are defined. In consequence, the lateral zones 76_{L}, 76_{R} of the separator sheet 33b are left free from the inner sheet 32 after the liquid-absorbent panel 33a and the separator sheet 33b have been bonded to each other. While the sections of the separator sheet 33b folded back onto itself are preferably not bonded together in the lateral zones 48_{L}, 48_{R}, these sections of the separator sheet 33b may be bonded together by adhesive or fusion-bonding in the front end zones 34 as well as in the rear end zones 36. While the non-woven fabric used to for the separator sheet 33b may be either liquid-pervious or liquid-impervious, it is rather preferable to use the non-woven fabric exhibiting liquid-perviousness lower than that of the inner sheet 32, more preferably, to use the liquid-impervious non-woven fabric. It is for the reason that the separator sheet 33b formed by such non-woven fabric will be able to prevent bodily fluids from moving from the liquid-absorbent panel 33a toward the wearer's skin even when the middle region 35 of the separator sheet 33b comes in contact with the liquid-absorbent panel 33a wetted with bodily fluids during use of the diaper 1. While a particular embodiment of the separator sheet 33b has been described above to comprise a pair of the non-woven fabric layers 46, 47, it is possible to form the separator sheet 33b by a single non-woven layer.

When the diaper 1' of Fig. 3 using the separator sheet 33b as has been described above is folded back along the transverse center line Q-Q, then the front and rear waist regions 12, 13 are bonded together along the respective lateral zones 15, 16 to obtain the diaper 1 of Fig. 1, the crotch region elastic members 37_{L}, 37_{R} contract, causing the middle region 35 of the separator sheet 33b to be spaced upward from the inner sheet 32 of the liquid-absorbent panel 33a in a middle region of the diaper 1 as viewed in the width direction B and thereby to bring the middle region 35 in contact with the wearer' s crotch region. At the same time, the liquid-absorbent panel 33a is bowed together with the covering chassis 2 below the middle region 35. Urine excreted by the wearer flows through the front opening 38 toward the liquid-absorbent panel 33a and feces excreted by the wearer moved through the rear opening 39 toward the liquid-absorbent panel 33a. Therefore, the wearer's skin would not be soiled with any amount of body waste at least between the external genital and the anus.

The separator sheet 33b is bonded to the inner sheet 32 along the lateral zones 48_{L}, 48_{R} by the adhesive regions 49_{L}, 50_{L}, 49_{R} and 50_{R} extending in the front-back direction A but not bonded to the inner sheet 32 along the lateral zones 76_{L}, 76_{R} in the middle region 35. Consequentially, the lateral zones 48_{L}, 48_{R} along the adhesive regions 49_{L}, 50_{L}, 49_{R}, 50_{R} are bowed together with the liquid-absorbent panel 33a as the liquid-absorbent panel 33a is bowed in the front-back direction A and the crotch region elastic members 37_{L}, 37_{R} contract. However, the lateral zones 76_{L}, 76_{R} as parts of the lateral zones 48_{L}, 48_{R} tend to become linear rather than following the liquid-absorbent panel 33a being bowed under contraction of the crotch region elastic members 37_{L}, 37_{R}. As a result, these lateral zones 76_{L}, 76_{R} are spaced upward from the liquid-absorbent panel 33a in the height direction C (See Fig. 2). At least an area of the middle region 35 on the longitudinal center line P-P is spaced upward from the liquid-absorbent panel 33a in close contact with the skin defined between the wearer's external genital and anus and thereby prevents the wearer' s skin from coming in contact with the liquid-absorbent panel 33a wetted with urine and/or feces and protects the wearer's skin from being soiled with urine and/or feces.

Such feature that the lateral zones 76_{L}, 76_{R} in the middle region 35 are free from the liquid-absorbent panel 33a along the non-adhesive regions 51_{L}, 51_{R}, i.e., the separator sheet 33b and the liquid-absorbent panel 33a are disconnected from each other in the width direction B of the diaper 1 leads to an advantageous effect as follows: Movement of the middle region 35 under the effect of the crotch region elastic members 37_{L}, 37_{R} is not affected by the liquid-absorbent panel 33a being bowed and sagging. Such effect promotes the movement of the middle region 35 getting near to the wearer's skin in comparison with the case in which the movement of the middle region 35 is more or less restricted by the liquid-absorbent panel 33a. In this way, the lateral zones 76_{L}, 76_{R} left free from the liquid-absorbent panel 33a serve as means for promote the movement of the middle region 35 to the wearer's skin. The feature of the crotch region elastic members 37 are attached under tension to the separator sheet 33b with the third segments 83_{L}, 83_{R} curved inward also elastically tightens the middle region 35 in the width direction B as these third segments 83_{L}, 83_{R} linearly contract. As an advantageous consequence, the middle region 35 is elastically tightened in the front-back direction A as well as in the width direction B so as to be kept in close contact with the wearer's skin substantially without the middle region 35 getting wrinkled and/or being folded during use of the diaper 1. The separator sheet 33b is formed with the U-shaped rear opening 39 having the bottom edge defined in the transverse middle of the separator sheet 33b and bonded to the inner sheet 32 along the lateral zones 48_{L}, 48_{R} and provided along the transversely opposite inner edges 73_{L}, 73_{R} of the rear opening 39 with the crotch region elastic members 37_{L}, 37_{R} extending in the front-back direction so as to keep these inner edges 73_{L}, 73_{R} in elastically tightened state. With such unique arrangement, the separator sheet 33b would not be caught by the cleft of the wearer's buttock and therefore the rear opening 39 would not be closed during use of the diaper 1 irrespective of the state in which the diaper 1 is on the wearer's body. In addition, the respective widths of the front opening 38 and the rear opening 39 are substantially not affected by expansion and contraction of the crotch region elastic members 37_{L}, 37_{R} used in the separator sheet 33b for the purpose as has been described above.

In the diaper 1, bodily fluids flowing in the width direction B on the upper surface of the separator sheet 33b surely flows back from spaces defined between the lateral zones 76_{L}, 76_{R} of the separator sheet 33b and the inner sheet 32 toward a central region of the liquid-absorbent panel 33a as viewed in the width direction B without leaking sideways from the diaper 1. While the respective components of the diaper 1 may be dimensioned within a wide range depending on whether the diaper 1 is for baby or for adult and depending on whether the diaper 1 is of the pant-type as illustrated or of the open-type, a dimension D₁ of the middle region 35 on the longitudinal center line P-P, i.e. , as measured in the front-back direction A (See Fig. 3 - Fig. 5) is preferably in a range of 20 to 70 mm in a typical example. A dimension D₂ of the respective lateral zones 76_{L}, 76_{R} (See Figs. 4 and 5) may be at least same as the dimension D₁ or larger than the dimension D₁ by 50 mm forward and rearward in the front-back direction A. The maximum dimensions D₃, D₄ of the front opening 38 and the rear opening 39 in the width direction B (See Fig. 4) are preferably in a range of 50 to 400 mm.

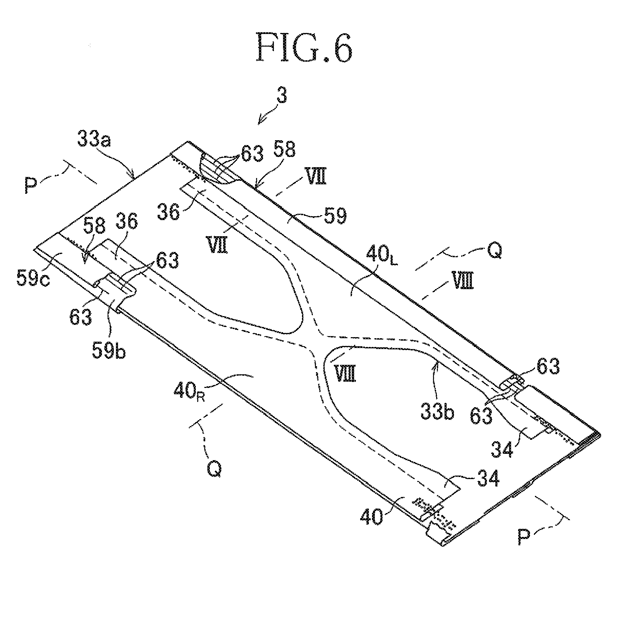

Fig. 6 is a view similar to Fig. 5, showing one exemplary embodiment of the bodily fluid-absorbent structure 3 used in the present invention. In the case of the bodily fluid-absorbent structure 3, the lateral zones 40_{L}, 40_{R} of the separator sheet 33b is provided on the inner side thereof with leak-barriers 58. Each of these leak-barriers 58 is formed by a non-woven fabric, preferably by a liquid-imperviousnon-woven fabric folded in a Z-shape or an inverted Z-shape.

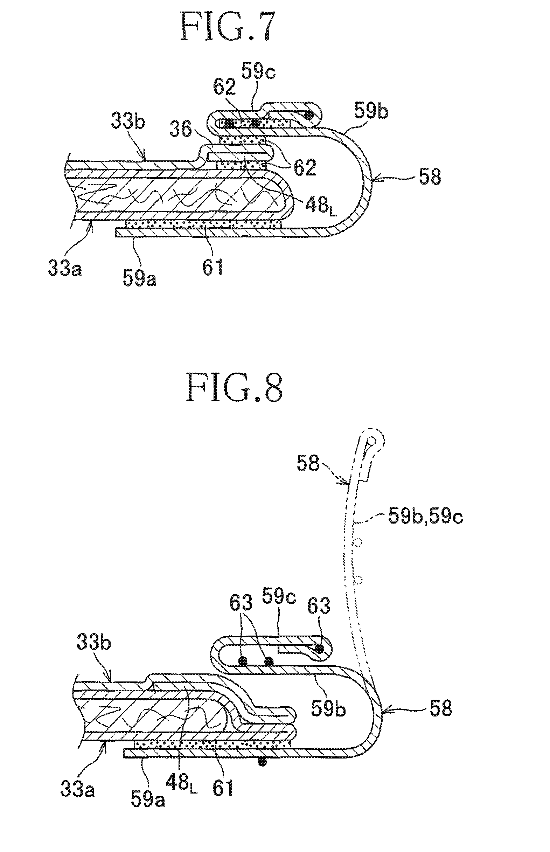

Fig. 7 is a sectional view taken along the line VII-VII in Fig. 6 and Fig. 8 is a sectional view taken along the line VIII-VIII in Fig. 6. Referring to Fig. 7, a bottom layer 59a of the leak-barrier 58 formed by folding a sheet of non-woven fabric or plastic film folded in a Z- or an inverted Z-shape is bonded to the bottom surface of the liquid-absorbent panel 33a by hot melt adhesive 61. An intermediate layer 59b and a top layer 59c of the Z- or inverted Z-shaped leak-proof barrier 58 are bonded to each other by hot melt adhesive 62 and the intermediate layer 59b is bonded to the inner surface of the separator sheet 33b at the front and rear end zones 34, 36 Referring to Fig. 8, the intermediate layer 59b and the top layer 59c extend in parallel to each other without being bonded to each other in the vicinity of the transverse center line Q-Q. Both the intermediate layer 59b and the top layer 59c are provided with elastic members 63 attached thereto under tension (See Fig. 6). When the diaper 1 using the bodily fluid-absorbent structure including such leak-proof barriers 58 is put into the state as shown by Fig. 1, the elastic members 63 contract in the front-back direction A and thereupon the intermediate layer 59b raises itself up together with the top layer 59c as indicated by imaginary line in Fig. 8, preventing sideways leak of bodily fluids from the bodily fluid-absorbent structure 3. The leak-proof barriers 58 serve also, in the diaper 1 of Fig. 1, to prevent bodily fluids from flow through gaps between the non-adhesive regions 51_{L}, 51_{R} of the liquid-absorbent panel 33a and the lateral edges 76_{L}, 76_{R} of the separator sheet 33b to the outside of the diaper 1.

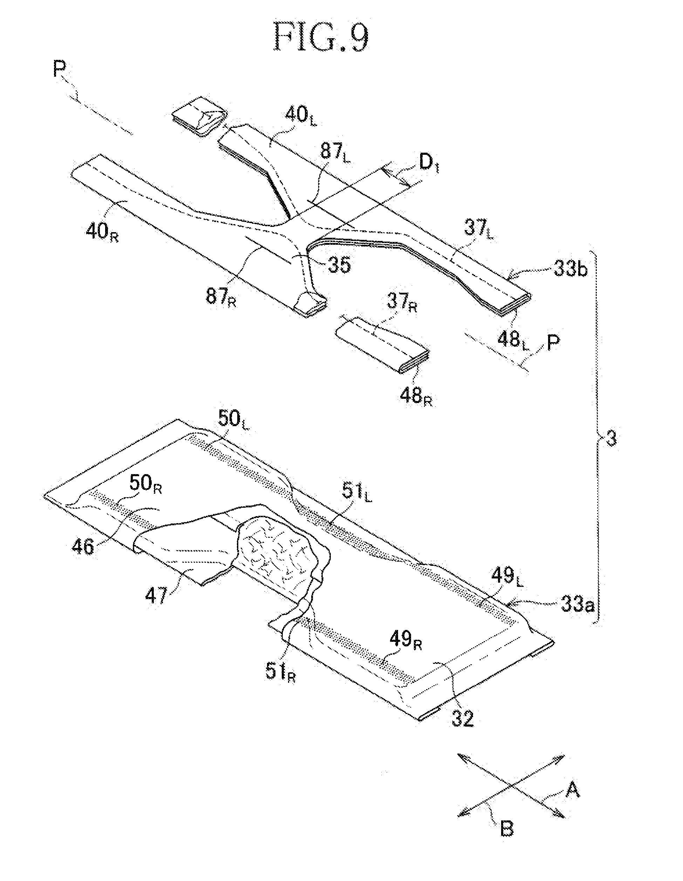

Fig. 9 is a view similar to Fig. 5, showing another exemplary embodiment of the present invention. The liquid-absorbent panel 33a constituting the bodily fluid-absorbent structure 3 in this embodiment is formed with adhesive regions 51_{L}, 51_{R} respectively contiguous to the adhesive regions 49_{L}, 50_{L} and 49_{R}, 50_{R} so that the adhesive regions 49_{L}, 50_{L} and 51_{L} and the adhesive regions 49_{R}, 50_{R} and 51_{R} may respectively form a pair of transversely opposite continuous adhesive regions. The separator sheet 33b is formed in each of lateral zones 40_{L}, 40_{R} with a slit 87_{L} or 87_{R} extending in the front-back direction A and through two non-woven fabric layers in the thickness direction. In this bodily fluid-absorbent structure 3 according to this embodiment, the lateral zones 48_{L}, 48_{R} of the separator sheet 33b are bonded to the inner sheet 32 of the liquid-absorbent panel 33a over the full length thereof. However, the slits 87_{L}, 87_{R} formed between the respective lateral zones 48_{L}, 48_{R} and the longitudinal center line P-P serve to alleviate a possibility that the lateral zones 48_{L}, 48_{R} bonded to the inner sheet 32 might restrict the middle region 35 of the separator sheet 33b against moving along the longitudinal center line P-P. Even if the lateral zones 48_{L}, 48_{R} af the separator sheet 33b as a whole follow bowing of the covering chassis 2 as the liquid-absorbent panel 33a of the diaper 1 bows as shown by Fig. 1, the middle region 35 of the separator sheet 33b under the contractile force of the elastic members 37_{L}, 37_{R} can move upward to the area of the wearer's skin defined between the wearer's external genital and anus while the slits 87_{L}, 87_{R} are opened. The slits 87_{L}, 87_{R} opened in this manner allow bodily fluids flowing on the upper surface of the separator sheet 33b in the width direction B to flow down toward the liquid-absorbent panel 33a with distance from the wearer's skin. The inventors found that each of the slits 87_{L}, 87_{R} may have a length at least same as the dimension D₁ of the middle region 35 so far as each of the slits extends short of the associated crotch region elastic member 37_{L} or 37_{R}. A distance in the width direction B between the longitudinal center line P-P and the respective slits 87_{L}, 87_{R} is preferably 20 mm or longer in the case of the baby diaper 1 and 40 mm or longer in the case of the adult diaper 1. These slits 87_{L}, 87_{R} also serve as means assisting a movement of the middle region 35 of the separator sheet 33b under the effect of the crotch region elastic members 37_{L}, 37_{R} to move toward the wearer's skin.

While the present invention has been described on the basis of the pant-type disposable diaper 1 as one the typical embodiments, the present invention is applicable also to the open-type disposable diaper.

## Claims

1. A disposable diaper (1) including a crotch region (11) having a front-back direction (A) and a width direction (B), a front waist region (12) extending forward from said crotch region and a rear waist region (13) extending rearward from said crotch region, said crotch region comprising a liquid-pervious inner sheet (32), a liquid-impervious outer covering sheet (7) and a bodily fluid-absorbent core (31) sandwiched between these two sheets, said disposable diaper further including a separator sheet (33b) extending in said front-back direction on the inner side of said inner sheet and formed in a front region thereof with a front opening (38) for passage of body waste and in a rear region thereof with a rear opening (39) for passage of body waste, said separator sheet having end regions (34, 36) opposite in said front-back direction being bonded to said inner sheet in said front waist region and said rear waist region, respectively, and said separator sheet including a middle region (35) defined between said front opening and said rear opening and adapted to be spaced upward from said inner sheet in said crotch region so as to prevent said inner sheet from coming in contact with a diaper wearer's skin, said disposable diaper being **characterized in that:**
said separator sheet has lateral zones (48) extending in said front-back direction and bonded to said inner sheet;
said separator sheet is provided on each side of a center line (P-P) bisecting a width of said diaper with at least a single elastic member (37) attached thereto under tension so as to extend along respective associated inner edges (71, 73) of said front opening and said rear opening in said front-back direction and to bow in said middle region toward said center line; and
said separator sheet is further formed along said lateral zones in said middle region or in areas defined between said lateral zones and said center line with means (87) assisting a movement of said middle region on said center line toward the diaper wearer's skin under contraction of said elastic members.

2. The diaper according to Claim 1 wherein said means are formed by leaving segments of respective said lateral zones of said separator sheet extending in said middle region not bonded to said inner sheet.

3. The diaper according to Claim 1 wherein said means are formed by providing said separator sheet with slits formed between respective said lateral zones and said center line, respectively, so as to extend in said front-back direction.

4. The diaper according to any one of Claims 1 through 3 wherein said front opening of said separator sheet is cut out from said separator sheet in said front region so as to describe a U-shape opening forward and said rear opening of said separator sheet is cut out from said separator sheet in said rear region so as to describe a U-shape opening rearward.
